# EUROPEAN PATENT APPLICATION

(11) **EP 3 925 641 A1**
(43) Date of publication of application: **22.12.2021**
(21) Application number: 21174273.9
(22) Date of filing: 18.05.2021
(51) Int. Cl.: A61M 3/02

(54) **IRRIGATION SYSTEM**

(30) Priority: 15.06.2020 SE 2050717
(71) Applicant: Dentsply IH AB, 431 21 Mölndal (SE)
(72) Inventor: LOVMAR, Martin, 431 69 Mölndal (SE)
(74) Representative: Lind Edlund Kenamets Intellectual Property AB

(57) **Abstract**

An irrigation system, for irrigation of a hollow body organ, comprises a reusable part and a disposable part. The reusable part comprises a liquid container with a first opening and a first connection interface. The disposable part comprises a protective cover comprising a tubular part, a one-way valve in the tubular part, and a protective skirt connected to and encircling the tubular part. The tubular part is provided with a second connection interface which is releasably connectable to the first connection interface.

## Description

### Technical Field

The present invention relates to an irrigation system, such as an enema system, a disposable protective cover for use in an irrigation/enema system, and a method for preparing an irrigation/enema system for use. The irrigation system is particularly intended for rectal irrigation, and is suitable for self-administration of an irrigation liquid.

### Background of the Invention

The present invention relates to an irrigation system for irrigation of a hollow body organ, such as the bowel. Administrating an irrigation liquid is a common medical procedure whereby liquid is injected into a bodily cavity, such as into the rectum, and sometimes also into the lower intestine, of a patient in order to induce bowel movement. The need for such a procedure typically arises in patients suffering from certain physical ailments in which voluntary bowel control is impaired or when the bowel needs to be cleaned before e.g. a coloscopy or a surgical operation. To this end, enema systems and the like may be used e.g. by people suffering from spinal cord injuries, spina bifida or multiple sclerosis. For such users, irrigation may improve quality of life by preventing constipation, reducing time spent for bowel emptying procedures, reducing fecal incontinence, and by increasing independency in general.

Irrigation is nowadays often performed outside medical attendance premises, such as in the patient's home, and is also often performed by the patient himself, i.e. by self-administration. It may also be necessary for the user to perform self-administration outside the home, such as in public restrooms and the like. It is therefore of importance that the irrigation system is of a limited size, and portable. Portability of the irrigation system is important to disabled persons who are not hospitalised or bed-ridden if they are to live as normal a life as possible. This is particularly important if they travel away from their home, for instance, to someone else's home or if they stay in a hotel. In this situation, they need to be able to deal with their bowel function easily.

Since the irrigation system is typically used quite frequently, such as several times each day, there is also a need to make the irrigation system as cost-effective as possible.

WO 2010/122537 and WO 2015/000488 disclose irrigation systems in which a liquid container is directly and releasably connected to a nozzle. After use, the nozzle may be discarded, or cleaned for further use. However, with such an irrigation system, there is a great risk that the hands of the user may be contaminated with the irrigation liquid and faeces during the procedure. In addition, the liquid container may also be contaminated during use, and would also need to be cleaned properly afterwards.

Thus, there is still a need for an irrigation system which can be made compact during storage, which is easy to use by self-administration, also for users having reduced dexterity, which can be used more efficiently, with reduced risk of contamination, and/or which can be produced in a cost-efficient way.

### Summary of the Invention

In view of the above-mentioned need, a general object of the present invention is to provide an irrigation system, a disposable protective cover for use in such an irrigation system, and a method for preparing an irrigation system for use, which alleviate the above-discussed problems of the prior art, and at least partly fulfil the above-discussed needs.

This and other objects are achieved with an irrigation system, a disposable protective cover and a method according to the appended claims.

According to a first aspect of the invention, there is provided an irrigation system, for irrigation of a hollow body organ, comprising a reusable part and a disposable part, the reusable part comprising a liquid container with a first opening and a first connection interface, and the disposable part comprising a protective cover comprising a tubular part, a one-way valve in the tubular part, and a protective skirt connected to and encircling the tubular part, wherein the tubular part is provided with a second connection interface which is releasably connectable to the first connection interface.

The hollow body organ can here be any cavity or canal in a human or mammal body having a body orifice, and e.g. includes an anal canal and an artificial anal canal, such as a stoma.

The first connection interface may be formed integrated with the first opening, or may alternatively be arranged as a separate element, arranged separated from the first opening. Similarly, the second connection interface may be formed by the tubular part itself, or be arranged as a separate part connected to the tubular part.

The tubular part may be formed as a generally cylindrical tube, e.g. having a circular or oval cross-section. However, it may also be formed as a conical or frusto-conical part, or the like.

The irrigation system can be made compact. The liquid container can be made relatively small, such as in the size of a toothpaste tube, or somewhat larger. The protective skirt can also be made of a very thin material, so that the protective cover can be compacted to a very limited size during storage. Thus, the whole irrigation system is very small, and can easily be carried around e.g. in a handbag or the like.

The protective skirt provides protection against contamination of the user's hand, holding the liquid container, during use. Further, the protective skirt also prevents contamination of the reusable part.

The one-way valve of the protective cover allows liquid flow in a direction out from the liquid container, and away from the reusable part, but prevents liquid flow in the other direction, i.e. towards the reusable part. Hereby, a further protection against contamination of the reusable part is obtained.

Thus, the protective cover provides a barrier between the insertable parts on the one hand, and the users hands and the reusable part on the other. Hereby, a very efficient protection against contamination of the reusable part is obtained, thereby obviating the need for any cleaning of it between uses. The protective cover may simply be disposed after use, and the reusable part stored for subsequent reuse.

For disposal, the protective skirt can be pulled up from the inside, and turned inside-out, over the tubular part for disposal. This enables handling of the protective cover only from the clean inside, making the entire process easy and without any risk for contamination. In the inside-out position, the protective cover may also be closed, by a knot or the like, providing a clean compartment with a clean exterior. The used protective cover can then be disposed in a waste bin, or be stored for later disposal.

Since the protective cover is releasably connected to the reusable part, the reusable part may be reused many times, by simply connecting it to a new protective cover each time. The reusable part may be arranged to be reusable for at least 10 times, such as at least 50 times, such as at least 100 times, or more. The reusability makes the entire system more compact, since the user may carry around several disposable parts, but only one reusable part. Further, it makes the irrigation system very cost-efficient, since the cost for the reusable part is spread out over multiple uses.

The liquid container can e.g. be filled beforehand and stored in a filled condition. In this case, the liquid container may be provided with a closable opening, such as with a removable lid or the like, to make it possible to handle it without the risk of spillage. This is e.g. of advantage when the irrigation system is to be used in a location where there is no access to tap water.

However, additionally or alternatively, the liquid container can easily be stored in an empty state, and then be filled e.g. with tap water, when it is to be used. If stored in an empty state, the container may also be arranged to be stored in a compacted, collapsed state. Such a state may e.g. be maintained by closing the opening with a removable lid or the like. Hereby, the container takes up less space when emptied, and will be returned into its full size when the opening is again opened, or alternatively when the container is filled with liquid.

The liquid container is preferably a flexible liquid container arranged to be emptied by applying pressure to it, such as by squeezing it, which is a simple process, which can easily be made with one hand only. The container is preferably dimensioned so that it fits within the grasp of a hand of an ordinary user.

However, larger liquid containers may also be used. In case the liquid container has a volume too large to be contained within a hand, the liquid container may also comprise one part, functioning as a squeezable pump, and a second part functioning as a reservoir. The parts may be integrated directly together, or be connected through a tubing or the like.

There are generally two types of irrigation of the rectal/anal/bowel. One is often referred to as transanal irrigation (TAI), where a probe is inserted a relatively long way, through the anal canal and into the rectum. In this type of irrigation, the probe is generally provided with a retention element, such as an inflatable balloon, which prevents withdrawal of the probe, and also seals the opening to enable a build-up of pressure in the anal canal and rectum, and to maintain the liquid within the bowel. The water softens the stool and causes peristaltic movements in the bowels, thereby pushing the stool towards the rectum to be evacuated. When the balloon is emptied, and the probe removed, the water and stool is released. In another type of irrigation, commonly referred to as enema or rectal irrigation, the probe is not as deeply inserted, and there is generally no build-up of pressure during the procedure. TAI is a more complicated procedure, but its effect lasts longer, whereas rectal irrigation is simpler, but normally only empties the lower part of the bowel.

The irrigation system of the present invention is useable for both TAI and rectal irrigation but is particularly suited for rectal irrigation. In one embodiment, the irrigation system is an enema system.

The tubular part, of the protective cover, has a length extending out from the protective skirt, in a direction away from the second connection interface. This length forms an insertable length, which is insertable into the anal canal of the user. The length is preferably at least long enough to bring the insertable end past the sphincter. The length may e.g. be in the range of 3-10 cm, and preferably within the range of 5-7 cm.

The protective skirt is preferably made of a flexible material and the tubular part is preferably made of a more rigid material. Thus, the protective skirt is more flexible than the tubular part, and the tubular part is more rigid than the protective skirt. The tubular part may e.g. be made of a plastic material, such as silicone, and may have a rigidity and stability enabling insertion into e.g. an anal opening, and essentially corresponding to those of a conventional rectal probe/catheter or urinary catheter. The protective skirt is preferably made of a very thin and flexible material, e.g. corresponding to plastic film used in freezer bags and the like.

The protective skirt is preferably connected at or adjacent to an end of the tubular part. Thus, the tubular part, along most of its length, extends out from the protective skirt, and preferably in a direction away from the second connection interface.

The protective skirt may be formed with a convex shape, such as forming a bell or a U in cross-section. However, the protective skirt may also be generally flat, and be draped over the reusable part and the user's hand by gravity.

The protective skirt may have a perimeter at a distance from the tubular part, wherein a distance from the tubular part to the perimeter exceeds a length of the liquid container. Hereby, it is ensured that the protective cover adequately protect the user's hand and the exterior of the reusable part from contamination during use.

Preferably, the distance between the perimeter and the tubular part is at least 10 cm, and more preferably at least 15 cm, and most preferably at least 20 cm.

The length around the perimeter is preferably long enough to accommodate the reusable part inside the disposable part, and preferably also to accommodate a hand of the user therein, and most preferably with some additional space, to allow sufficient room for manipulation and manoeuvring. The length around the perimeter may be at least 30 cm, and preferably at least 40 cm, and most preferably at least 50 cm.

The disposable part may further comprise a retention element arranged on the tubular part. The retention element is hereby part of the disposable part of the irrigation system, and is arranged on a part of the disposable part forming the insertable element. The function of the retention element is to provide a certain resistance for withdrawal of the inserted part, thereby maintaining it in its inserted position until it is deliberately pulled out. This is of advantage, since it e.g. hinders that the inserted part falls out, should the user e.g. by accident lose his/her grip on the liquid container. The retention element also prevents irrigation liquid and faeces to fall out during withdrawal of the inserted part, and also during the irrigation procedure.

The retention element is preferably arranged as a flexible member extending outwards from the tubular part. The flexible member is preferably a non-inflatable member, and preferably extends laterally outwards from the tubular part. The retention element may e.g. be in the form of a disc encircling the tubular part. The disc may be generally flat in the expanded configuration, but may also be shaped as a bell, umbrella or chalice or the like, with a curved surface directed upwards or downwards. However, other retention elements are also feasible, such as petals extending out from the tubular part, a bulbous part, e.g. filled with air, foam, gel or the like.

The retention element is preferably configured to be deformed from an initial, large, expanded configuration, prior to insertion into the anal canal or other hollow organ, to a collapsed, small configuration during advancement into the anal canal or other hollow organ, and then back into the initial configuration upon full advancement of the retaining element into the hollow organ, such as when advanced passed the sphincter. In the inserted expanded configuration, the retention element serves the function of retaining the inserted part in position. During withdrawal, the retaining element is again collapsed, and thereby serves as a sealing of the hollow organ during the withdrawal, thereby preventing leakage of liquid and faeces.

The retention element is preferably arranged at or in the vicinity of an end of the tubular part being opposite to the second connection interface. The retention element is further preferably arranged at a distance from the second connection interface. However, embodiments without any retention element are also feasible.

The tubular part may comprise an outlet opening centrally, in the longitudinal extension of the tubular part. However, the tubular part may also have one or more outlet openings arranged laterally, in the side wall of the tubular part. In one embodiment, the tubular part may have a closed end, and preferably a rounded closed end, and with outlet opening(s) arranged only in the side wall.

The reusable part may further comprise an insertion stop member arranged in the vicinity of the first opening, said insertion stop member extending transversely to a longitudinal direction of the opening. The insertion stop member may e.g. be arranged as a disc, encircling the opening of the liquid container. Alternatively, the insertion stop member may extend only over a certain part around the tubular member, such as forming one or more circular sectors. Alternatively, the insertion stop member may extend as arms in two, three or more directions.

The insertion stop member serves the purpose of prohibiting too deep insertion of the insertable part into the anal canal or the like. At the same time, the insertion stop member also functions as a handle, facilitating insertion and other manipulation of the irrigation system during the irrigation procedure.

In an alternative embodiment, the insertion stop member may instead be arranged as part of the disposable part, and e.g. be arranged on the tubular part.

In one embodiment, the liquid container comprises only one opening, i.e. the first opening. In this case, this opening is used both for filling the container with liquid prior to use, and for discharging the liquid during the irrigation procedure.

However, in other embodiments, the liquid container further comprises a second opening, preferably arranged in an end of the liquid container which is opposite to the first opening, and two one-way valves, wherein a first of said one-way valves is adapted to allow fluid to exit the liquid container through said first opening, and a second of said one-way valves is adapted to allow fluid to enter the liquid container through said second opening. In such an embodiment, the liquid container may be flexible and function as a bladder/bellow pump. In an empty state, the second opening may be arranged in contact with a liquid, such as in the outflow from a water tap, or when immersed in water. The container may then be squeezed to remove air from the container through the first one-way valve. When the squeezing pressure is removed, the container will relax to an expanded state, by sucking in liquid through the second one-way valve. When filled, the tubular part may be inserted into the site of irrigation, such as into the anal canal. When the container is then again squeezed, the liquid will be expelled from the container, through the first opening, for irrigation.

Alternatively, the one-way valve of the disposable part may serve as the first one-way valve once arranged on the reusable part. For such use, the liquid container may only be provided with one, i.e. the second, one-way valve.

Thus, in such embodiments, the liquid container may function as a bulb pump, with the liquid holding compartment of the container being made of a resilient, squeezable material, which retains its shape when unloaded. By squeezing the liquid holding compartment, the fluid is pumped out through the first opening, and when the squeezing is relieved, the pumping compartment retains its original shape, thereby sucking in fluid through the second opening.

However, it is also possible to use other types of liquid containers, such as more rigid liquid containers, and to discharge the liquid by a separate pump, such as a manually operated pump or an electrically operated pump.

The first and second connection interfaces are preferably relatively simple to engage and disengage from each other. Further, the connection formed by the interfaces is preferably stable enough to maintain the disposable and reusable parts properly connected during both insertion, irrigation and withdrawal. In an embodiment, the first and second connection interfaces may form one of a threaded connection, a bayonet connection and a friction fit connection.

The first opening may be arranged directly at an end of e.g. a bulbous liquid container. However, the first opening may also be arranged on a neck formed in the liquid container. Hereby, the neck forms a long or short tube connecting the opening to the rest of the container. Such a neck may be used to form the interface to connect to the tubular part, e.g. by means of a friction fit. It may also be used to maintain a certain distance from the major part of the container and the insertable/inserted part, to ease manipulation.

In one embodiment, the neck may be dimensioned and arranged to extend into a part of, and preferably an essential part of, and most preferably essentially the entire, tubular part when the disposable part is arranged on the reusable part. In such a situation, the insertable part will be formed both by the tubular part of the disposable part, and, at least to some extent, by the neck of the liquid container. The neck may then serve both the function of forming a connection between the disposable and reusable parts, and to provide rigidity and stability to the insertable part.

The protective skirt is preferably compactable to a compacted storage state, and expandable to an expanded use state. In the compacted storage state, the protective cover may be arranged in an outer package, such as a thin plastic container, a small paper box or the like, to ensure that it maintains its compacted state. However, alternatively, the compacted state may be maintained by other means, such as a rubber band, adhesive and the like.

At least part of the disposable part may be made of a material which breaks down or disintegrates when immersed in water. Hereby, the disposable part may be flushed into a toilet after use, and then disintegrate or break down. This makes disposal even simpler.

The irrigation system is preferably arranged for rectal or bowel irrigation. To this end, the tubular part is preferably arranged and dimensioned to be insertable into an anal canal of a user. However, it is also feasible to use the irrigation system for irrigation of other hollow body organs.

According to another aspect of the invention, there is provided a disposable protective cover for use in an irrigation system for irrigation of a hollow body organ, comprising a tubular part, a one-way valve in the tubular part, and a protective skirt connected to and encircling the tubular part, wherein the tubular part is provided with an interface adapted to be releasably connected to an opening interface of an opening of a reusable liquid container.

This aspect provides similar functions, features and advantages as discussed above with reference to the first embodiment.

According to yet another aspect of the invention, there is provided a method for preparing an irrigation system for irrigation of a hollow body organ for use, the method comprising:
providing a reusable part comprising a liquid container with a first opening and a first connection interface;
providing a disposable part comprising a protective cover comprising a tubular part, a one-way valve in the tubular part, and a protective skirt connected to and encircling the tubular part, wherein the tubular part is provided with a second connection interface; and
releasably connecting the disposable part to the reusable part by releasably connecting the first connection interface and the second connection interface.

This aspect provides similar functions, features and advantages as discussed above with reference to the first embodiment.

These and other aspects of the invention will be apparent from and elucidated with reference to the embodiments described hereinafter.

### Brief description of the drawings

For exemplifying purposes, the invention will be described in closer detail in the following with reference to embodiments thereof illustrated in the attached drawings, wherein:
Figs. 1a-1f are perspective side views of various exemplary embodiments of a reusable part for use in an irrigation system;
Figs. 2a-2c are perspective view (Fig. 2a) and schematic cross-sectional views (Figs. 2b and 2c) of various exemplary embodiments of a disposable part for use in an irrigation system;
Fig. 3 is a perspective view of an assembled irrigation system, comprising a reusable part and a disposable part; and
Fig. 4 is a schematic view illustrating a disposable part after use, prepared for disposal.

### Detailed description of preferred embodiments

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled addressee. Like reference characters refer to like elements throughout. The disclosed irrigation system is here described as a system for rectal or bowel irrigation. However, the irrigation system may also be used for irrigation of other hollow body organs.

Embodiments of an irrigation system comprise a reusable part and a disposable part, which are connected together for use. After use, the parts are disconnected, and the disposable part may be disposed, whereas the reusable part may be stored for reuse.

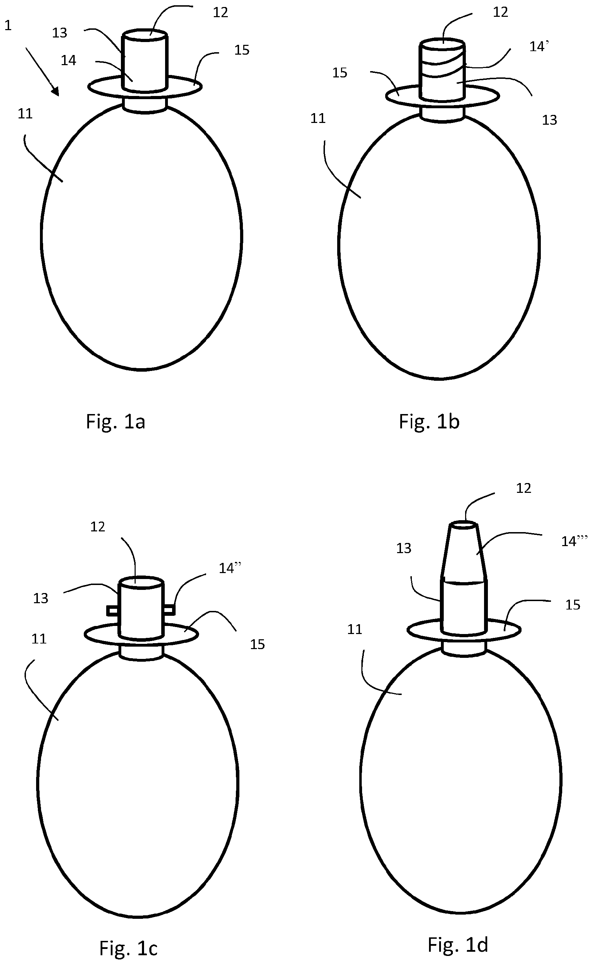

Embodiments of the reusable part 1 are illustrated in Figs. 1a-f. The reusable part comprises a liquid container 11 with a first opening 12 and a first connection interface 14.

The liquid container 11 may be flexible and may e.g. be formed as a bulb, with a generally elliptical cross-section, in order to make it easy to squeeze. However, other shapes are also feasible, such as a container in the form of a cuboid, a cone or a cylinder. However, other types of liquid containers and pump arrangements are also feasible. The liquid container may e.g. be sized to fit the grasp of a hand, such as being the size of an apple or an orange. The container may e.g. have an interior volume of 0.05-1 litre, and preferably in the range of 0.1-0.7 litre, and most preferably in the range of 0.1-0.5 litre. However, the container may also have a larger interior volume.

The liquid container is provided with at least one opening, the first opening 12. This opening is useable as an outlet for the irrigation liquid from the container during irrigation. However, in case there is only one opening, as in the exemplary embodiment of Fig. 1a, this opening may also function as an inlet, for filling the container with irrigation liquid, such as water, prior to use.

The liquid container can e.g. be filled beforehand, and stored in a filled condition. In this case, the liquid container may be provided with a closable opening, such as with a removable lid or the like (not shown), to make it possible to handle it without the risk of spillage.

However, additionally or alternatively, the liquid container can easily be stored in an empty state, and then be filled e.g. with tap water, when it is to be used. In this case, and where the liquid container is flexible, a removable lid or the like may be used to maintain the empty container in a collapsed, compacted state.

The opening 12 may be arranged on a tube connected to the rest of the container, thereby forming a neck 13 in the container.

A connection interface - the first connection interface 14 - is further provided on the container, and preferably on the neck/tube 13. The connection interface is releasably connectable to a corresponding second connection interface on the disposable part.

The first connection interface 14 may be arranged as an internal surface within the neck 13, to engage with an external surface of a tubular part of the disposable part, to form a connection with this, e.g. through a friction fit. Such an embodiment is illustrated in Fig. 1a.

However, the first connection interface 14' may alternatively be arranged on an external surface of the neck, for co-operation with an internal surface of a tubular part of the disposable part. Such an embodiment is illustrated in Fig. 1b. In this embodiment, the interface is further provided with threads, to provide a threaded connection. Here, the first connection interface is provided with threads on an external surface, whereas the second connection interface (not shown) comprises corresponding threads on an internal surface. However, the first connection interface may also comprise internal threads, and the second connection interface comprise external threads.

Alternatively, the first connection interface 14" may be provided with radially extending pins, on an external surface, to provide a bayonet mount/bayonet connection with the second connection interface. The second connection interface is in this case provided with a shaped slot for receiving the pins. Such an embodiment is illustrated in Fig. 1c. Naturally, the first connection interface may instead comprise the shaped slot, and the second connection interface may comprise the pins.

In the embodiment shown in Fig. 1a, the first connection interface connected to the second connection interface by a friction fit between an internal surface of the first connection interface and an external surface of the second connection interface. However, the friction fit connection may also be the other way around, as in the embodiment illustrated in Fig. 1d. Here, the friction fit connection is formed between an external surface of the first connection interface 14"', and a corresponding internal surface of the second connection interface. To facilitate the connection, and also enhance the connection, a part of the neck 13 of the liquid container may have a conically tapering form, thereby facilitating insertion of it into the corresponding tubular part of the disposable part, and also to make the connection stronger.

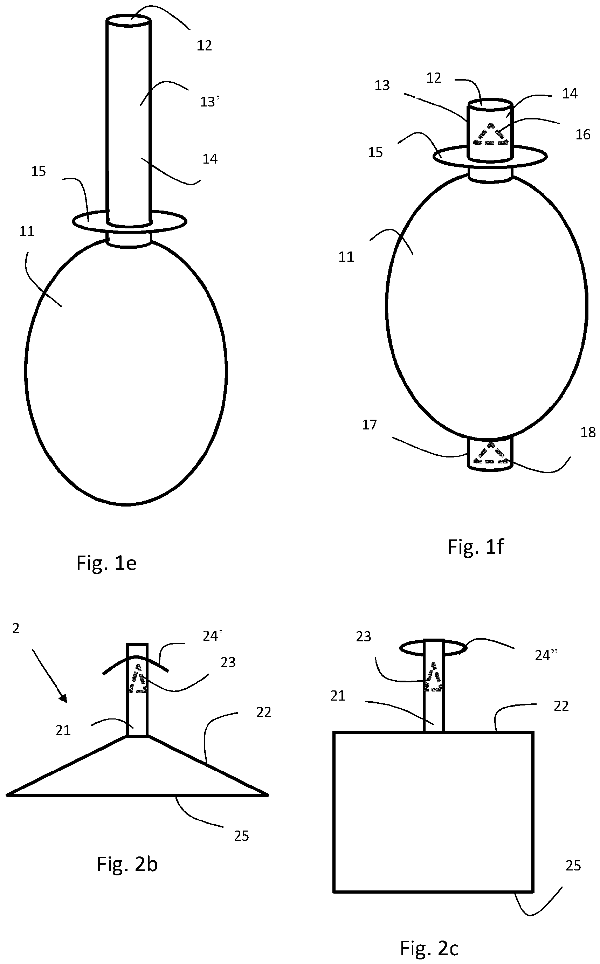

In the exemplary embodiments of Figs. 1a-d, the neck 13 is relatively short. However, it is also possible to use a container 11 without a neck, and to arrange the first connection interface directly in or on the wall of the container of the container. However, it is also feasible to use a longer neck 13', as in the exemplary embodiment of Fig. 1e. In such an embodiment, the neck may be arranged to extend farther into the tubular part of the disposable part when connected, or even over essentially the whole length of the tubular part. The neck may then serve both the function of forming a connection between the disposable and reusable parts, and to provide rigidity and stability to the insertable part. However, additionally, or alternatively, such a longer neck 13' may also be used to provide a further distance between the disposable part and the liquid container when connected together.

In the exemplary embodiments of Figs. 1a-1e, the container is provided with a single opening 12. In these cases, this opening is used both for filling the container with liquid prior to use, and for discharging the liquid during the irrigation procedure.

However, in other embodiments, the liquid container further comprises a second opening 17, preferably arranged in an end of the liquid container 11 which is opposite to the first opening 12. Such an embodiment is illustrated in Fig. 1f. Here, two one-way valves may be provided, wherein a first one-way valve 16 is adapted to allow fluid to exit the liquid container through said first opening 12, and a second one-way valve 18 is adapted to allow fluid to enter the liquid container through the second opening 17. Thus, the liquid container here functions as a bladder/bellow pump. In an empty state, the second opening may be arranged in contact with a liquid, such as in the outflow from a water tap. The container may then be squeezed to remove air from the container through the first one-way valve. When the squeezing pressure is removed, the container will relax to an expanded state, by sucking in liquid through the second one-way valve. When filled, the tubular part may be inserted into the site of irrigation, such as into the anal canal. When the container is then again squeezed, the liquid will be expelled from the container, through the first opening, for irrigation. The container may be made of a resilient, squeezable material, which retains its shape when unloaded.

In case the disposable part, with its one-way valve, is arranged on the reusable part, this one-way valve may serve as the first one-way valve. In such an embodiment, the liquid container only needs to have one, the second, one-way valve.

The one-way valve may e.g. be realized as a duck-bill valve.

The reusable part may further comprise an insertion stop member 15, e.g. arranged in the vicinity of the first opening 12, as in the embodiments of Figs. 1a-d and f, when a short neck is provided. However, in case the neck is longer, and in particular if it is to be inserted deeply into the tubular part of the disposable part, as in Fig. 1e, the insertion stop member 15 may be arranged at some distance away from the first opening 12.

The insertion stop member 15 preferably extends transversely to a longitudinal direction of the opening/neck. However, the insertion stop member may also be angled, curved or shaped to extend partly in a transversal direction, and partly in an upward or downward direction. The insertion stop member may e.g. be arranged as a disc, encircling the opening of the liquid container. Alternatively, the insertion stop member may extend only over a certain part around the tubular member, such as forming one or more circular sectors. Alternatively, the insertion stop member may extend as arms in two, three or more directions.

The insertion stop 15 member serves the purpose of prohibiting too deep insertion of the insertable part into the anal canal or the like. At the same time, the insertion stop member also functions as a handle, facilitating insertion and other manipulation of the irrigation system during the irrigation procedure.

Alternatively, the insertion stop member may be arranged on the disposable part, and preferably on the tubular part.

The above-discussed reusable part may be reused multiple times, and may each time be combined with a disposable part.

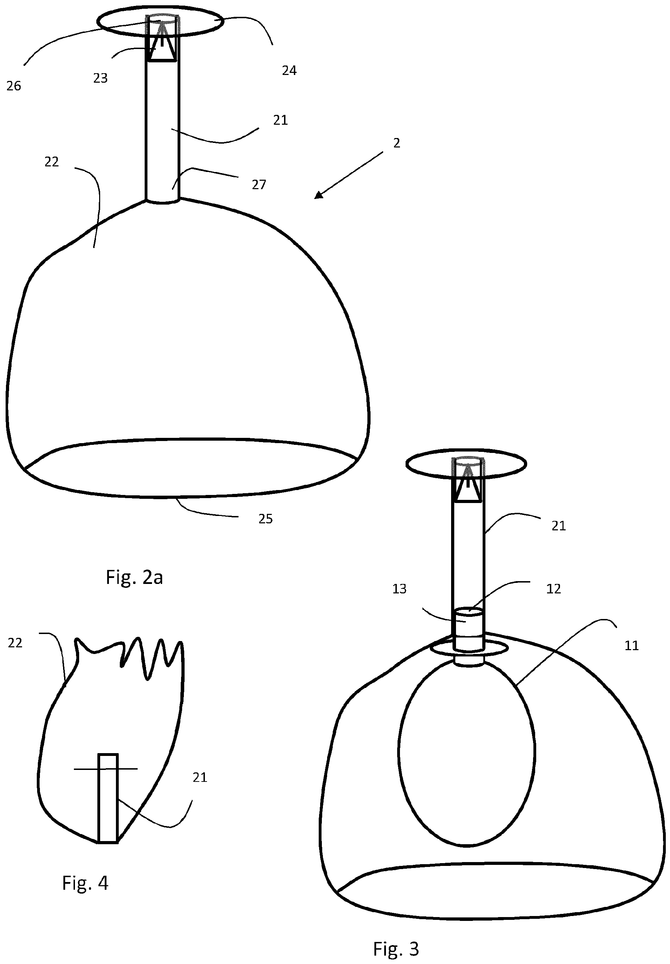

Exemplary embodiments of the disposable part are illustrated in Figs. 2a-c. The disposable part 2 comprises a protective cover comprising a tubular part 21, a one-way valve 23 in the tubular part, and a protective skirt 22 connected to and encircling the tubular part 21. The tubular part is provided with the second connection interface 27, as discussed in the foregoing, which is releasably connectable to the first connection interface. As discussed in the foregoing, the second connection interface is preferably complementary to the first connection interface, e.g. to form a threaded connection, a bayonet connection or a friction fit connection. Further, the second connection interface may preferably be arranged on an internal surface within the tubular part. However, it is also feasible to have a tubular part extending both upwardly and downwardly past the protective skirt, and to arrange the second connection interface on an external surface thereof.

The tubular part has in the foregoing been disclosed as an essentially cylindrical tube, with a centrally arranged outlet opening. However, the tubular part may also have laterally arranged outlet openings, arranged in the sidewall of the tubular part. In one embodiment, the tubular part may have a closed end, and preferably a rounded closed end, and only one or more lateral openings through the sidewall.

Further, the tubular part may be formed as a cylindrical tubular part with a generally uniform cross-section. However, the tubular part may also have a non-uniform shape, such as a tapering form. For example, the tubular part may be shaped as a cone. Such a conically shaped tubular part may also be compactable into a compact storage state, and extended and elongated into a longer state when arranged on the reusable part.

Still further, the one-way valve has in the foregoing been disclosed as being arranged in the forward, insertable part of the tubular part. However, the one-way valve may be arranged in any location along the length of the tubular part, and may e.g. be arranged adjacent to, or in the vicinity of, the second connection interface.

The one-way valve 23 of the protective cover allows liquid flow in a direction out from the liquid container, and away from the reusable part, but prevents liquid flow in the other direction, i.e. towards the reusable part. Hereby, a further protection against contamination of the reusable part is obtained.

The one-way valve may e.g. be realized as a duck-bill valve. Such a valve may comprise a unit made in one piece, e.g. by molding, and made of a flexible material, such as silicone. The valve may comprise a tube with an outlet opening part formed in the shape of a duck bill, forming a slit shaped opening at the end. The valve formed by the duck bill end will allow passage of liquid coming through the tubular part from the liquid container but will prevent passage of liquid in an opposite direction. However, many other types of one-way valves, which are per se known, may also be used.

The tubular part 21 of the protective cover has a length extending out from the protective skirt 22, in a direction away from the second connection interface. This length forms an insertable length, which is insertable into the anal canal of the user. The length is preferably at least long enough to bring the insertable end past the sphincter. The length may e.g. be in the range of 3-10 cm, and preferably within the range of 5-7 cm.

The protective skirt 22 is preferably made of a flexible material and the tubular part 21 is preferably made of a more rigid material. Thus, the protective skirt 22 is more flexible than the tubular part 21, and the tubular part 21 is more rigid than the protective skirt 22. The tubular part 21 may e.g. be made of silicone, and may have a rigidity and stability enabling insertion into e.g. an anal opening, and essentially corresponding to those of a conventional rectal probe/catheter or urinary catheter. The protective skirt 22 is preferably made of a very thin and flexible material, e.g. corresponding to plastic film used in freezer bags and the like.

The protective skirt and the tubular part may be made of plastic material. However, other materials are also feasible, such as cellulose-based materials.

The disposable part may be made fully or partly in a transparent material, as in the illustrative examples, in order to facilitate assembly of the irrigation system.

The protective skirt 22 is preferably connected at or adjacent to an end of the tubular part. Thus, the tubular part 21 may, along most of its length, extend out from the protective skirt 22, and preferably in a direction away from the second connection interface 27.

The protective 22 skirt may be formed with a convex shape, such as forming a bell or a U in cross-section, as in the illustrative example of Fig. 2a. However, the protective skirt may also be generally flat, and extend in a plane, or in a relatively flat cone, such as in the illustrative example of Fig. 2b. In such an embodiment, the protective skirt will, in use, fall down over the reusable part and the user's hand, due to gravity, and be folded and draped to form a general shape resembling the one of Fig. 2a. However, other shapes are also feasible, such as a rectangular shape, i.e. the shape of a conventional freezer bag, as in the illustrative example of Fig. 2c. Other shapes are also feasible, e.g. various 3-dimensional shapes, such as the shape of a cylinder, cuboid and the like.

The protective skirt 22 may have a perimeter 25 at a distance from the tubular part 21, wherein the distance from the tubular part to the perimeter at all places exceeds a length of the liquid container. Thus, put differently, the protective skirt is preferably arranged to completely cover the reusable part, and preferably also to completely cover the hand of the user used for manipulation of the container. Preferably, the distance between the perimeter and the tubular part is, at a minimum, at least 10 cm, and more preferably at least 15 cm, and most preferably at least 20 cm.

The length around the perimeter 25 is preferably long enough to accommodate the reusable part inside the disposable part, and preferably also to accommodate a hand of the user therein, and most preferably with some additional space, to allow sufficient room for manipulation and manoeuvring. The length around the perimeter 25 may be at least 30 cm, and preferably at least 40 cm, and most preferably at least 50 cm.

The disposable part may further comprise a retention element 24 arranged on the tubular part 21. The retention element 24 is hereby part of the disposable part of the irrigation system, and is arranged on a part of the disposable part forming the insertable element. The function of the retention element is to provide a certain resistance for withdrawal of the inserted part, thereby maintaining it in its inserted position until it is deliberately pulled out.

The retention element 24 is preferably arranged as a flexible member extending outwards from the tubular part. The flexible member is preferably a non-inflatable member, and preferably extends laterally outwards from the tubular part. The retention element may e.g. be in the form of a disc encircling the tubular part, as in the illustrative example of Fig. 2a. The disc may be generally flat in the expanded configuration, but may also be shaped as a bell, umbrella or chalice or the like, with a curved surface directed upwards or downwards. Such a retention element 24' is illustrated in Fig. 2b. However, other retention elements are also feasible, such as petals extending out from the tubular part, and the like. It is also feasible to use a bulbous part, e.g. filled with air, foam, gel or the like. Such a retention element 24" is illustrated in Fig. 2c.

The retention element 24, 24', 24" is preferably configured to be deformed from an initial, large, expanded configuration, prior to insertion into the anal canal or other hollow organ, to a collapsed, small configuration during advancement into the anal canal or other hollow organ, and then back into the initial configuration upon full advancement of the retaining element into the hollow organ, such as when advanced passed the sphincter. In the inserted expanded configuration, the retention element serves the function of retaining the inserted part in position. During withdrawal, the retaining element is again collapsed, and thereby serves as a sealing of the hollow organ during the withdrawal, thereby preventing leakage of liquid and faeces.

The retention element is preferably arranged at or in the vicinity of an end of the tubular part, at or close to the opening 26, being opposite to the second connection interface 27.

However, embodiments without any retention element are also feasible.

The protective skirt 22 is preferably compactable to a compacted storage state, and expandable to an expanded use state. In the compacted storage state, the protective cover may be arranged in an outer package, (not shown) such as a thin plastic container, a small paper box or the like, to ensure that it maintains its compacted state. However, alternatively, the compacted state may be maintained by other means, such as a rubber band, adhesive and the like.

Fig. 3 illustrates the irrigation system in an assembled state, where the disposable part is connected to the reusable part.

Upon use, the liquid container is filled with a suitable irrigation liquid. The irrigation liquid can be any liquid which is capable of irrigation the body cavity of interest, such as water, hypertonic aqueous salt solutions, solutions or suspensions of cathartic agents, such as bisacodyl or phenolphthalein, and mineral oil. The container may be prefilled. However, if it is to be refilled, ordinary tap water is normally preferred as the irrigation liquid.

By use of the present invention, rectal irrigation can be carried out by the following steps:
- The liquid container is filled with a liquid, e.g. regular tap water;
- The disposable part is unpacked (if packed) and unfolded;
- The reusable part and the disposable part are connected together - however, this step may be made before or after the unpacking and unfolding of the disposable part;
- The tubular part of the disposable part is inserted into the anal canal;
- The liquid container is operated, e.g. by being compressed, to transfer irrigation liquid from the container to the tubular part for irrigation;
- When irrigation is completed, the tubular part is removed from the anal canal; and
- The disposable part is disconnected from the reusable part.

The protective skirt 22 provides protection against contamination of the user's hand, holding the liquid container, during use. Further, the protective cover prevents contamination of the reusable part. Thus, there is no need to clean the reusable part after use.

The disposable part may be immediately discarded. For disposal, the protective skirt can be pulled up from the inside, and turned inside-out, over the tubular part for disposal. This enables handling of the protective cover only from the clean inside, making the entire process easy and without any risk for contamination. In the inside-out position, the protective cover may also be closed, by a knot or the like, providing a clean compartment with a clean exterior. Such a disposable part, turned inside-out for disposal, is illustrated in Fig. 4. The used protective cover can then be disposed in a waste bin, or be stored for later disposal.

The disposable part may also be turned inside-out while still connected to the reusable part, to facilitate handling.

At least part of the disposable part may also be made of a material which breaks down or disintegrates when immersed in water. Hereby, the disposable part may be flushed into a toilet after use, and then disintegrate or break down. This makes disposal even simpler.

The person skilled in the art realizes that the present invention is not limited to the preferred embodiment. For example, the various examples of retention elements, connection interfaces, and the like, mentioned in the different embodiments above may be combined in all possible ways. Further, the one-way valves may be of various types, and the parts may be produced by various materials.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. An irrigation system, for irrigation of a hollow body organ, comprising a reusable part and a disposable part, the reusable part comprising a liquid container with a first opening and a first connection interface, and the disposable part comprising a protective cover comprising a tubular part, a one-way valve in the tubular part, and a protective skirt connected to and encircling the tubular part, wherein the tubular part is provided with a second connection interface which is releasably connectable to the first connection interface.

2. The irrigation system of claim 1, wherein the protective skirt is made of a flexible material and the tubular part is made of a more rigid material.

3. The irrigation system of claim 1 or 2, wherein the protective skirt is connected at or adjacent to an end of the tubular part.

4. The irrigation system of any one of the preceding claims, wherein the protective skirt has a perimeter at a distance from the tubular part, wherein distance from the tubular part to the perimeter exceeds a length of the liquid container.

5. The irrigation system of claim 4, wherein a length around said perimeter is at least 30 cm, and preferably at least 40 cm, and most preferably at least 50 cm.

6. The irrigation system of any one of the preceding claims, wherein the disposable part further comprises a retention element arranged on said tubular part.

7. The irrigation system of any one of the preceding claims, wherein the reusable part further comprises an insertion stop member arranged in the vicinity of the first opening, said insertion stop member extending transversely to a longitudinal direction of the opening.

8. The irrigation system of any one of the preceding claims, wherein the liquid container further comprises a second opening, preferably arranged in an end of the liquid container which is opposite to the first opening, and two one-way valves, wherein a first of said one-way valves is adapted to allow fluid to exit the liquid container through said first opening, and a second of said one-way valves is adapted to allow fluid to enter the liquid container through said second opening.

9. The irrigation system of any one of the preceding claims, wherein the first and second connection interfaces form one of a threaded connection, a bayonet connection and a friction fit connection.

10. The irrigation system of any one of the preceding claims, wherein the first opening is arranged in a neck formed in the liquid container.

11. The irrigation system of any one of the preceding claims, wherein the protective skirt is compactable to a compacted storage state, and expandable to an expanded use state.

12. The irrigation system of any one of the preceding claims, wherein at least part of the disposable part is made of a material which breaks down or disintegrates when immersed in water.

13. The irrigation system of any one of the preceding claims, wherein the tubular part is arranged and dimensioned to be insertable into an anal canal of a user.

14. A disposable protective cover for use in an irrigation system for irrigation of a hollow body organ, comprising a tubular part, a one-way valve in the tubular part, and a protective skirt connected to and encircling the tubular part, wherein the tubular part is provided with an interface adapted to be releasably connected to an opening interface of an opening of a reusable liquid container.

15. A method for preparing an irrigation system for irrigation of a hollow body organ for use, the method comprising:
providing a reusable part comprising a liquid container with a first opening and a first connection interface;
providing a disposable part comprising a protective cover comprising a tubular part, a one-way valve in the tubular part, and a protective skirt connected to and encircling the tubular part, wherein the tubular part is provided with a second connection interface; and
releasably connecting the disposable part to the reusable part by releasably connecting the first connection interface and the second connection interface.
